# EUROPÄISCHE PATENTANMELDUNG

(11) **EP 3 750 591 A1**
(43) Veröffentlichungstag der Anmeldung: **16.12.2020**
(21) Anmeldenummer: 20179748.7
(22) Anmeldetag: 12.06.2020
(51) Int. Cl.: A61N 1/16

(54) **SCHUTZEINRICHTUNG GEGEN ELEKTROMAGNETISCHE WELLEN UND FELDER**

(30) Priorität: 14.06.2019 DE 102019116324; 23.12.2019 DE 102019135778
(71) Anmelder: Vivobase GmbH, 72124 Pliezhausen (DE)
(72) Erfinder: Seipel, Jochen, 72124 Pliezhausen (DE)
(74) Vertreter: Sattler de Sousa e Brito, Clara

(57) **Zusammenfassung**

Vorrichtung zum Schutz gegen elektrische und/oder magnetische Felder, insbesondere elektromagnetische Felder, umfassend eine Schaltung und ein mit der Schaltung gekoppeltes Feld, wobei die Schaltung wenigstens zwei Schutzelemente aufweist, wobei ein Schutzelement wenigstens einen Filmkondensator und wenigstens eine Diode umfasst. Die Schaltung gibt naturähnliche Wellenpakete, erzeugt von den Dioden und Kondensatoren durch Spontanentladungen in die Umgebung und das elektrische Feld der Stromleitung ab, wobei die Signale den elektrischen Entladungen von AIS entsprechen. Des Weiteren wird auch ein Verfahren zum Betreiben einer solchen Vorrichtung angegeben.

## Beschreibung

Die Erfindung betrifft eine Vorrichtung zum Schutz gegen elektrische und/oder magnetische Felder, insbesondere elektromagnetische Felder, und ein Verfahren zum Betreiben einer solchen Vorrichtung.

### HINTERGRUND DER ERFINDUNG

Belastungen durch elektromagnetische Wellen und Felder für Lebewesen können auftreten durch: Netzleitungen, Induktionskochfelder, Elektromotoren, Wechselrichter, Radiowellen und Fernsehgeräte, funkgesteuerte Uhren, und Mobiltelefonnetze wie 3G,4G,5G etc. Jedes Gerät, das drahtlos Informationen überträgt, verursacht Elektrosmog.

Durch Elektrosmog werden die natürlich vorkommenden Ladungen in der Natur zerrüttet bzw. gestört. Der Mensch erzeugt ebenso Felder und hat sich der Natur angepasst, Für ihn sind die Felder essentiell. Durch die Magnetkristalle, die im menschlichen Gehirn vorhanden sind, ist der Mensch in der Lage die feinsten Ladungen oder Schwankungen wahrzunehmen. Diese Schwankungen werden im Unterbewusstsein als Stress oder Belastung wahrgenommen. Das kann zu einem geschwächten Immunsystem und einem ständig angespannten und ruhelosen Bewusstseinszustand führen.

Die technischen Felder, die Elektrosmog auslösen, besitzen gegenüber natürlichen Feldern eine extrem konstante Periodizität und eine hohe Ordnung, wohingegen natürliche Felder eine gewisse Aperiodizität und Unordnung besitzen. Lebewesen weisen in ihrer elektromagnetischen Zusammensetzung ebenfalls ein gewisses Verhältnis von geordneten und ungeordneten Feldern auf. Durch die Exposition mit Elektrosmog erfährt der Mensch ein Übermaß an geordneten Feldern. Empfindliche Personen können darauf mit Stress und Missempfindung reagierten. Abhilfe kann die Zufuhr naturähnlicher Wellenpakete bieten, die das Missverhältnis zwischen Ordnung und Unordnung wieder ausgleichen können.

### ZUSAMMENFASSUNG DER ERFINDUNG

Es ist Aufgabe der vorliegenden Erfindung, eine Schutzeinrichtung gegen elektromagnetische Wellen und Felder anzugeben, so dass in einfacher Art und Weise das gestörte Gleichgewicht natürlich vorkommender Ladungsfelder und den Feldern des Menschen und der anderen Lebewesen, bzw. Verhältnis das zwischen Ordnung und Unordnung durch die Erzeugung naturähnlicher Felder wieder hergestellt wird.

Die erfindungsgemäße Lösung der Aufgabe erfolgt durch die Merkmale der unabhängigen Ansprüche. Vorteilhafte Weiterbildungen der Erfindung ergeben sich aus den Unteransprüchen.

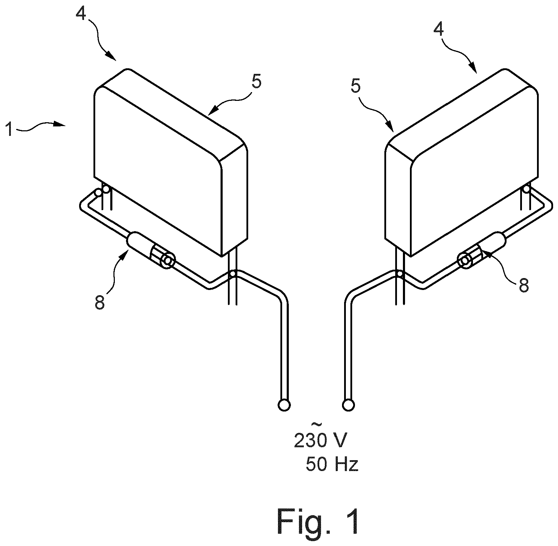

Der Erfindung liegen folgende Kenntnisse zugrunde. Wie in Figur 1 gezeigt besteht eine Schaltung 1 aus zwei sogenannten Vivobase-Elementen 4. Ein Vivobase-Element 4 besteht aus einem Polypropylen-Filmkondensator 5 und einer Sperrdiode 8. Der Kondensator 5 ist nur an einer Seite an das Netz angeschlossen. Die Diode 8 wird parallel zum Kondensator 5 angebracht, die Sperrschicht zeigt zum Netzanschluss. Ein Element wird dann an den spannungsführenden Leiter und ein Element an den Null-Leiter angeschlossen. Der Kondensator 5 hat eine Kapazität von 0,022µF und ist bis 1000V DC belastbar. Zwischen den Kondensatoren 5 entsteht ein Feld, welches das Rauschen der Dioden 8 in die Umgebung überträgt. Durch den unipolaren Anschluss des Vivobase-Elementes 4 entsteht ein Potentialgefälle über dem Kondensator zu seiner Umgebung, das zu seiner Aufladung führt. Überschreitet die Aufladung des Kondensators 5 die Sperrspannung der Sperrdiode 8 kommt es zu einer Spontanentladung des Kondensators, die ähnlich einer Gewitterentladung ein Wellenpaket im Bereich von 100 Hz bis 18 kHz entstehen lässt. Durch eine gegenläufige Anordnung der Dioden 8 in den Vivobase-Elementen 4 können beide Halbwellen der ansteuernden Wechselspannung ausgenutzt werden.

Die erfindungsgemäße Lösung der Aufgabe erfolgt durch eine Vorrichtung zum Schutz gegen elektrische und/oder magnetische Felder, insbesondere elektromagnetische Felder, umfassend eine Schaltung und ein mit der Schaltung gekoppeltes Feld. Die Schaltung weist wenigstens zwei Schutzelemente auf. Ein Schutzelement umfasst wenigstens einen Filmkondensator und wenigstens eine Diode. Die Schaltung lässt durch Spontanentladungen Wellenpakete entstehen, die der natürlichen, atmosphärischen Impulsstrahlung (AIS) ähneln. Die Schaltung gibt das Rauschen der Dioden, sowie die aus den Resonanzeigenschaften des Kondensators und der Diode entstehende Frequenz, in das elektrische Feld ab. Die Kombination der Signale entspricht den elektrischen Entladungen wie bei einer Sferics.

Durch die erfindungsgemäßen Maßnahmen wird eine Vorrichtung ausgebildet, die sich durch eine Schutzeinrichtung gegen elektromagnetische Felder auszeichnet, wobei dies naturähnliche elektromagnetische Wellen/Felder und elektrostatische Wellen/Felder (Elektrosmog) sind, für alle Lebewesen zu jeder Zeit und an jedem Ort. Der Effekt der Vorrichtung, im Folgenden auch Vivobase genannt, stellt das gestörte Gleichgewicht, natürlich vorkommender Ladungsfelder und den Felder des Menschen und der anderen Lebewesen, wieder her. Vivobase macht sich einen Effekt der Natur zu eigen.

Durch den unipolaren Anschluss der Schutzelemente entsteht gegenüber der Umgebung ein Potentialgefälle, das den Kondensator bei ansteigender Amplitude der Steuerspannung auflädt. Erreicht die Aufladung die Schwellenspannung der Diode, wird der Kondensator kurzgeschlossen und es entsteht hierdurch eine Spontanentladung. Dieser Vorgang wiederholt sich bei der nächsten Halbwelle der Netzfrequenz/Anregungsfrequenz mit gleicher Polarität. Eine gegenläufige Anordnung der Dioden erlaubt die Ausnutzung beider Halbwellen der Netzfrequenz/Anregungsfrequenz. Die Spontanentladungen erzeugen Wellenpakete, die den natürlichen, atmosphärischen Impulsstrahlungen (AIS) ähneln.

Zwischen den Schutzelementen entsteht ein elektrisches Feld. In dieses Feld wird durch die Schaltung, welche Grundschaltung genannt wird, ein Signal abgegeben, dass aus dem Grundrauschen der Diode besteht und der Resonanzfrequenz aus Kondensator und Diode, die dadurch entsteht, dass der Kondensator bei ansteigender Amplitude aufgeladen wird und bei Erreichen der Schwellenspannung der Diode, kurzgeschlossen wird. Dieser Vorgang wiederholt sich bis die Amplitude der Netzfrequenz/Anregungsfrequenz wieder unter die Schwellenspannung fällt. Diese Signalkombination entspricht den elektrischen Entladungen, wie bei Sferics.

Die Sferics oder auch atmosphärischen Impulsstrahlungen (AIS) entstehen durch elektrische Entladungen der Atmosphäre in großer Höhe um den gesamten Globus und sind daher für das Auge unsichtbar. Sie verursachen das impulshafte Auftreten elektromagnetischer Wellen natürlichen Ursprungs innerhalb der Erdatmosphäre.

Die Sferics entsteht durch elektrische Entladungen wie sie beispielsweise in einer Wolke stattfinden. Im Gegensatz zu einem Blitz bei einem Gewitter, ist die Sferics für das Auge unsichtbar. Sfericserscheinungen können ein bis zwei Stunden bevor ein Gewitter aufzieht auf Messgeräten erkennbar sein.

Unter Sferics (kurz für Englisch atmospheric; bisweilen auch atmosphärische Impulsstrahlung oder AIS) versteht man das impulshafte Auftreten elektromagnetischer Wellen natürlichen Ursprungs innerhalb der Erdatmosphäre. Sferics oder AIS sind sehr kurz dauernde, oft nur aus wenigen Schwingungen bestehende Wellenpakete, die durch Ladungsverschiebungen entstehen. Es handelt sich um sogenannte gedämpfte Schwingungen. Die Frequenzen liegen zwischen 3 und 100 kHz.

Die Sfericserscheinungen oder AIS können bei auch gutem Wetter auftreten, haben dann jedoch andere Eigenschaften wie bei schlechtem Wetter, beispielsweise Gewitter. Die Unterschiede sind beispielsweise die Höhe der Amplituden, die Impulsdauer und die Impulsfolgefrequenz sowie die Frequenz der Entladungen/Verschiebungen.

Mit anderen Worten, die Vorrichtung schafft ein der Natur nachempfundenes Frequenzspektrum oder Rauschen, welches den Mensch in seinem richtigen Rhythmus hält durch die Zufuhr naturähnlicher Wellenpakte.

Die erzeugten Wellenpakte erreichen außerdem eine Polarisation der Wassermoleküle im Körper von Menschen und anderen Lebewesen, so dass weniger Elektrosmog aufgenommen wird und sich auch ein positiver Effekt bei thermischen Belastungen von Elektrosmog einstellt.

Oder anders formuliert: Durch die Dipoleigenschaft des Wassermoleküls und das erzeugte elektrische Feld, schaffen die Vorrichtung es außerdem die im menschlichen Körper vorhanden Wassermoleküle zu polarisieren und den Mensch so vor der thermischen Belastung von Elektrosmog zu schützen.

Die Vorrichtung sieht in einer Weiterentwicklung vor, dass die Diode eine Shottky-Diode ist. Vorteilhaft dabei ist, dass erstens: Die Frequenzen der Vivobase-Geräte hängen von den Resonanzeigenschaften der Vivobase-Elemente und deren Ansteuerung ab. Es wurde erkannt, dass Shottky-Dioden mit einer kürzeren Refraktärzeit als normalen Dioden das Spektrum zu höheren Frequenzen verschieben, weil diese bei 50 Hz öfter dh. schneller schalten können als die normalen Dioden. Zweitens: Shottky-Dioden besitzen ein stärkeres Grundrauschen als normale Dioden.

Eine andere Weiterentwicklung sieht vor, dass die Diode eines der Schutzelemente gedreht zur Diode eines der anderen Schutzelemente am Kondensator angeschlossen wird. Gemäß einem Beispiel wird die Diode eines der Schutzelemente gedreht zur Diode eines der anderen Schutzelemente am Kondensator angeschlossen. Ein Schutzelement übernimmt die positive Halbwelle und ein anderes Schutzelement die negative Halbwelle. Bislang wurden die Dioden im Null-Leiterweg oder am spannungsführenden Leiter auf dieselbe Art und Weise an den Kondensator angeschlossen.

In einer anderen Weiterentwicklung ist vorgesehen, dass der Filmkondensator ein SMD-Polypropylen-Filmkondensator ist. Gemäß einem Beispiel ist der Filmkondensator ein SMD-Polypropylen-Filmkondensator. Bislang wurde angenommen, dass die gewünschten Resonanzeigenschaften, verursacht durch die Kapazität, Bauform, Materialeigenschaft und Größe, nur mit dem einen bisher verwendeten Kondensator, erreicht werden. Durch die kürzere Refraktärzeit der Shottky-Dioden kann auch dieser Bautyp die gewünschten Resonanzeigenschaften erreichen. Somit kann die Schaltung deutlich platzsparender gebaut werden. Damit ist die Möglichkeit gegeben den Schutz, durch Vivobase, in jeder Situation anzubieten.

Um die Vorrichtung noch weiter zu verbessern ist in einer Weiterentwicklung vorgesehen, dass die Schaltung mit verschiedenen Frequenzen und/oder Frequenzprogrammen betrieben wird. Gemäß einem Beispiel wird die Schaltung mit verschiedenen Frequenzen betrieben. Die eingestellte Frequenz und die daraus resultierende Impulsdauer steuert die Intensität des Feldes. Somit können sie das Empfinden des Menschen gegenüber dem Feld des Vivobase, in der Eingewöhnungsphase, positiv oder negativ beeinflussen.

In einer anderen Weiterentwicklung ist vorgesehen, dass die Schaltung eine Verstärkerschaltung aufweist, die mit den wenigstens zwei Schutzelementen gekoppelt ist. Gemäß einem Beispiel weist die Schaltung eine Verstärkerschaltung auf, die mit den wenigstens zwei Schutzelementen gekoppelt ist. Frequenzen und Signalformen werden damit über die Verstärkerschaltung wieder an die Kondensator-Dioden-Kombination geführt. Diese Ausführungsform wird auch Home-Variante oder Corporate-Variante genannt, weil sie mittels Netzspannung von 230 V betrieben wird. Zuallererst wird die Spannung gleichgerichtet und im Anschluss neu moduliert, sodass andere Frequenzen bzw. Frequenzprogramme und auch Signalformen, genutzt werden können.

Gemäß einem Beispiel sind die Dioden oder Shottky-Dioden parallel zum Kondensator geschaltet. Zwei Dioden werden mit dem spannungsführenden Leiter und zwei Dioden werden mit dem Neutralleiter verbunden.

Gemäß einem Beispiel weist die Schaltung eine Frequenzgeneratorschaltung auf, die über eine Kaskade mit den wenigstens zwei Schutzelementen gekoppelt ist. Die Frequenzschaltung wird über eine Spannungsversorgung angetrieben, beispielsweise ein Akku oder eine 12V Gleichspannungsquelle. Über eine Kaskade wird die Frequenzgeneratorschaltung an die Schutzelemente, umfassend den Kondensator und die Diode, geführt. Bei dieser Schaltung befindet sich jeweils ein Schutzelement am Neutralleiter und an der Phase. Diese Ausführungsform wird auch Mobile-Variante genannt und wird mit so kleinen Dimensionen gebaut, dass man die Platine in ein Armband, Schlüsselanhänger oder Hundemarke/Halsband einbetten kann.

Eine andere Weiterentwicklung sieht vor, dass die Vorrichtung ein Gehäuse mit einem Display aufweist. Auf dem Display kann eine Uhr, der Zustand des Akkus oder der Betriebsstatus ablesbar sein. Außerdem können verschiedene Programme auswählbar sein, mit denen die Schaltung mit verschiedenen Frequenzen betrieben werden kann.

Gemäß eines weiteren Aspekts der Erfindung wird ein Verfahren zum Betreiben der Vorrichtung angegeben. Das Verfahren umfasst: Erzeugen von naturähnlichen Wellenpaketen durch Spontanentladung der Kondensatoren über Dioden, die den natürlichen AIS ähneln.

Ein weiteres Verfahren umfasst: Steuern der Schaltung zum Abgeben des Rauschens der Dioden, sowie die aus den Resonanzeigenschaften des Kondensators und der Diode entstehende Frequenz, in das elektrische Feld; und Kombinieren der Signale, wobei die Kombination der Signale den elektrischen Entladungen wie bei einer Sferics entspricht.

Die Schaltung soll mit verschiedenen Frequenzen betrieben werden können. Die eingestellte Frequenz kann das Empfinden des Menschen gegenüber dem Feld des Vivobase positiv oder negativ beeinflussen. Die Vivobase Geräte werden durch die Schaltung, mit verschiedenen Frequenzen bzw. Frequenzprogramm betrieben werden können, da in der Natur nicht nur eine Form von AIS oder Sferics vorkommt.

Vorteilhafte Weiterbildungen der Erfindung ergeben sich aus den Unteransprüchen.

### KURZE BESCHREIBUNG DER FIGUREN

Weitere Einzelheiten der Erfindung sind den Ausführungsbeispielen zu entnehmen, die im Folgenden anhand der Figuren beschrieben wird. Es zeigen:
Figur 1: Schaltung mit zwei Vivobase-Elementen;
Figur 2: Schaltung einer Vorrichtung zum Schutz gegen elektrische und/oder magnetische Felder, insbesondere elektromagnetische Felder; und
Figur 3: weitere Schaltung einer Vorrichtung zum Schutz gegen elektrische und/oder magnetische Felder, insbesondere elektromagnetische Felder.

### AUSFÜHRLICHE BESCHREIBUNG DER FIGUREN

Im Folgenden werden exemplarische Ausführungsformen der Erfindung unter Bezugnahme auf die beiliegenden Zeichnungen beschrieben:

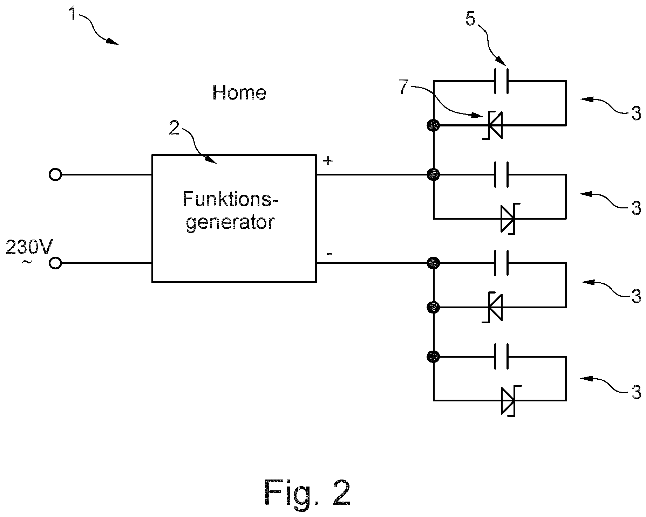

Figur 2 zeigt eine Grundschaltung 1 einer Vorrichtung (Vivobase) zum Schutz gegen elektrische und/oder magnetische Felder, insbesondere elektromagnetische Felder. Diese Art der Grundschaltung 1 ist beispielsweise zur Nutzung in einem Haus (Home) oder in einem Unternehmen (Corporate) vorgesehen.

Die Schaltung 1 weist vier Schutzelemente 3 auf. Ein Schutzelement 3 umfasst in diesem Beispiel jeweils einen Filmkondensator 5 und eine Diode 7 (der besseren Übersicht halber sind diese Bezugszeichen nur bei einem Schutzelement angegeben).

Die Schaltung 1 gibt die Wellenpakete der Vivobase-Elemen Schutzelemente 3 in die Umgebung und das elektrische Feld der Stromleitung ab. Die Wellenpakete ähneln den natürlichen AIS.

Anders formuliert: Die Schaltung 1 gibt das Rauschen der Dioden 7, sowie die aus den Resonanzeigenschaften des Kondensators 5 und der Diode 7 entstehende Frequenz, in das elektrische Feld ab. Die Kombination der Signale entspricht den elektrischen Entladungen wie bei einer Sferics.

Die Grundschaltung 1 wird mit 230V Netzspannung betrieben. Im Funktionsgenerator 2 wird die Spannung gleichgerichtet und im Anschluss neu moduliert, sodass andere Frequenzen bzw. Frequenzprogramme und auch Signalformen, genutzt werden können. Diese Frequenzen und Signalformen werden dann über eine Verstärkerschaltung an die Kondensator-Dioden-Kombination 3 geführt.

Verwendet werden in diesem Beispiel Schottky-Dioden 7 parallel zum Kondensator 5: zwei am spannungsführenden Leiter und zwei am Neutralleiter. Zwischen den Elementen 3 kann sich eine Glimmlampe mit einem Vorwiderstand befinden.

Der Betriebszustand der Vorrichtung wird beispielsweise über eine dreifarbige LED abgelesen.

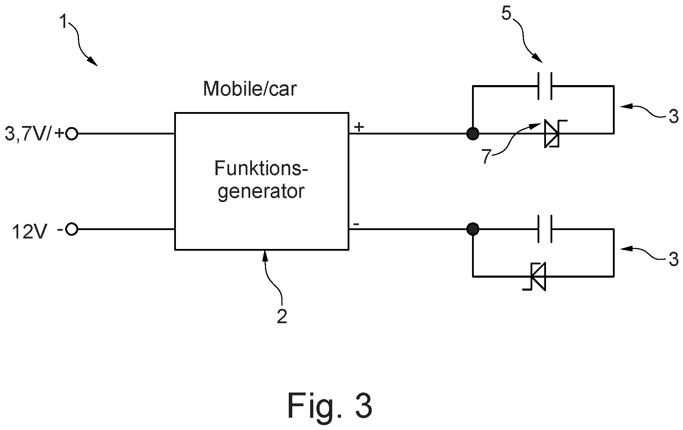

In Figur 3 ist eine weitere Grundschaltung 1 einer Vorrichtung (Vivobase) zum Schutz gegen elektrische und/oder magnetische Felder, insbesondere elektromagnetische Felder, gezeigt. Diese Art der Grundschaltung 1 ist beispielsweise zur mobilen Nutzung (Mobile) oder in einem Fahrzeug (Car) vorgesehen.

Die Spannungsversorgungen sind, ein Akku 3,7V oder eine 12V Gleichspannungsquelle. Über die Spannung wird eine Frequenzgeneratorschaltung 2 angetrieben und diese über eine Kaskade an die zwei Schutzelemente 3 geführt, jeweils einen Kondensator 5 und Diode 7 umfassend.

Bei dieser Schaltung 1 für ein Vivobase-Mobile befindet sich jeweils ein Kondensator-Dioden Paar 3 am Neutralleiter und an der Phase. Hier werden wieder die gleichen Schottky-Dioden wie beim Beispiel in Figur 2 (Home) verwendet, wobei die Kondensatoren 5 durch SMD Kondensatoren 5 mit den gleichen Eigenschaften ersetzt sind.

Die Spannungsversorgung kann ein Lithiumionen-Akku dessen Spannung wechselgerichtet, verstärkt und dann an die zwei Elemente 3 geführt wird. Der spannungsführende Leiter an ein Element 3 und der Neutralleiter an das andere Element 3. Die Schaltung 1 wird mit 25Hz betrieben.

Die Schaltung 1 der Vivobase-Car Variante ist wie bei Vivobase-Mobile, außer dass die Spannungsversorgung eine USB Schnittstelle, statt dem Akku ist.

Die Mobile-Variante, die in Figur 3 gezeigt ist, wird vom Aufbau so klein gehalten, dass man die Platine in ein Armband, Schlüsselanhänger oder Hundemarke/Halsband einbetten kann.

In manchen Beispielen verfügen diese Gehäusevarianten über ein Display. Auf dem Display kann eine Uhr, der Zustand des Akkus und der Betriebsstatus ablesbar sein. Außerdem können verschiedene Programme auswählbar sein, mit denen die Schaltung 1 mit verschiedenen Frequenzen betrieben wird. Die Platinen in der jeweiligen Variante Vivobase-Corporate oder Vivobase-Animal sind gleich wie bei Vivobase-Home, lediglich die Gehäuse unterscheiden sich.

Der Betriebszustand dieser Car-Variante kann auch über eine dreifarbige LED ablesbar sein. Dazu kann ein entsprechendes Programm fest eingestellt sein.

Es ist zu beachten, dass die in diesem Dokument beschriebenen Verfahren, Vorrichtungen und Systeme sowohl alleine als auch in Kombination mit anderen in diesem Dokument beschriebenen Verfahren, Vorrichtungen und Systemen verwendet werden können. Des Weiteren können jegliche Aspekte der in diesem Dokument beschriebenen Verfahren, Vorrichtung und Systemen in vielfältiger Weise miteinander kombiniert werden. Insbesondere können die Merkmale der Ansprüche in vielfältiger Weise miteinander kombiniert werden.

Die Erfindung ist anhand der Zeichnungen und der obigen Beschreibung ausführlich beschrieben. Diese Erfindung kann jedoch in vielen verschiedenen Formen ausgeführt werden und sollte nicht als auf die hier dargelegten Ausführungsformen begrenzt ausgelegt werden; vielmehr sind diese Ausführungsformen vorgesehen, damit diese Offenbarung gründlich und vollständig ist, und decken den Schutzumfang der Erfindung für einen Fachmann vollständig ab. Die Terminologie, die in der ausführlichen Beschreibung der in den beiliegenden Zeichnungen dargestellten Ausführungsformen verwendet wird, soll für die Erfindung nicht einschränkend sein. In den Zeichnungen beziehen sich gleiche Zeichen auf gleiche Elemente.

## Patentansprüche

1. Vorrichtung zum Schutz gegen elektrische und/oder magnetische Felder, insbesondere elektromagnetische Felder, umfassend
- eine Schaltung und
- ein mit der Schaltung gekoppeltes Feld,
- wobei die Schaltung wenigstens zwei Schutzelemente aufweist
- wobei ein Schutzelement wenigstens einen Filmkondensator und wenigstens eine Diode umfasst,
- **dadurch gekennzeichnet ist, dass** die Schaltung aus Diode und Kondensator naturähnliche Wellenpakete durch Spontanentladung erzeugt und in die Umgebung und das elektrische Feld der Stromleitung abgibt, wobei die Signale den elektrischen Entladungen von AIS ähneln.

2. Vorrichtung, insbesondere nach Anspruch 1, zum Schutz gegen elektrische und/oder magnetische Felder, insbesondere elektromagnetische Felder, umfassend
- eine Schaltung und
- ein mit der Schaltung gekoppeltes Feld,
- wobei die Schaltung wenigstens zwei Schutzelemente aufweist
- wobei ein Schutzelement wenigstens einen Filmkondensator und wenigstens eine Diode umfasst,
**dadurch gekennzeichnet, dass** die Schaltung das Rauschen der Dioden, sowie die aus den Resonanzeigenschaften des Kondensators und der Diode entstehende Frequenz, in das elektrische Feld abgibt, wobei die Kombination der Signale den elektrischen Entladungen wie bei einer Sferics entspricht.

3. Vorrichtung nach Anspruch 1 oder 2, **dadurch gekennzeichnet, dass** die Diode eine Shottky-Diode ist.

4. Vorrichtung nach Anspruch 1 bis 3, **dadurch gekennzeichnet, dass** die Diode eines der Schutzelemente gedreht zur Diode eines der anderen Schutzelemente am Kondensator angeschlossen wird.

5. Vorrichtung nach einem der vorherigen Ansprüche, **dadurch gekennzeichnet, dass** der Filmkondensator ein SMD-Polypropylen-Filmkondensator ist.

6. Vorrichtung nach einem der vorherigen Ansprüche, **dadurch gekennzeichnet, dass** die Schaltung mit verschiedenen Frequenzen und/oder Frequenzprogrammen betrieben wird.

7. Vorrichtung nach einem der vorherigen Ansprüche, wobei die Schaltung eine Verstärkerschaltung aufweist, die mit den wenigstens zwei Schutzelementen gekoppelt ist.

8. Vorrichtung nach Anspruch 7, **dadurch gekennzeichnet, dass** die Dioden oder Shottky-Dioden parallel zum Kondensator geschaltet sind.

9. Vorrichtung nach einem der Ansprüche 1 bis 6, **dadurch gekennzeichnet, dass** die Schaltung eine Frequenzgeneratorschaltung aufweist, die über eine Kaskade mit den wenigstens zwei Schutzelementen gekoppelt ist.

10. Vorrichtung nach einem der vorherigen Ansprüche, **dadurch gekennzeichnet, dass** die Vorrichtung ein Gehäuse mit einem Display aufweist.

11. Verfahren zum Betreiben einer Vorrichtung nach einem der Ansprüche 1 bis 9, wobei das Verfahren umfasst:
Steuern der Schaltung zum Abgeben von naturähnlichen Wellenpaketen mittels der Dioden und Kondensatoren in die Umgebung und das elektrische Feld von Stromleitungen.
